(19) Europäisches Patentamt
European Patent Office
Office européen des brevets

(11) Numéro de publication: **0 090 712 B1**

# (12) FASCICULE DE BREVET EUROPEEN

(45) Date de publication du fascicule du brevet: **19.06.85**

(51) Int. Cl.⁴: **C 07 C 21/18**

(21) Numéro de dépôt: **83400569.6**

(22) Date de dépôt: **18.03.83**

## (54) Bis-(perfluoroalkyl)-1,2-éthènes ramifiés, leur préparation et leur utilisation comme transporteurs d'oxygène.

(30) Priorité: **26.03.82 FR 8205165**

(43) Date de publication de la demande: **05.10.83 Bulletin 83/40**

(45) Mention de la délivrance du brevet: **19.06.85 Bulletin 85/25**

(84) Etats contractants désignés: **AT BE CH DE FR GB IT LI NL SE**

(56) Documents cités:
**US - A - 2 880 247**

**CHEMICAL ABSTRACTS, vol. 98, 1983, page 383, no. 113650d, Columbus Ohio USA; M.LE BLANC et al.: "A strategy for the synthesis of pure, inert perfluoroalkylated derivatives designed for blood substitution"**
**TETRAHEDRON, vol. 29, août 1973, pages 2411-2414, Pergamon Press, Oxford GB; G. SANTINI et al.: "The reaction of perfluoroalkylcopper compounds with 1-bromo-1-perfluoroalkylethylenes"**
**TETRAHEDRON, vol. 30, décembre 1974, pages 4197-4200, Pergamon Press, Oxford GB; F. JEANNEAUX et al.: "Synthèse de tetrakis(perfluoroalkyl)-1,2,3,4 butadienes-1,3 par duplication des bis(perfluoroalkyl)-1,2 iodoéthènes en présence de cuivre: une nouvelle série de transporteurs de gaz dissous pour usages biologiques"**

(73) Titulaire: **ATOCHEM, 12/16, allée des Vosges, F-92400 Courbevoie (FR)**

(72) Inventeur: **Riess, Jean Georges, Les Glaînes, F-06950 Falicon (FR)**
Inventeur: **Jeanneaux, François, 135 Avenue Saint-Lambert, F-06100 Nice (FR)**
Inventeur: **Le Blanc, Maurice, 10 Avenue de Brancolor, F-06100 Nice (FR)**
Inventeur: **Lantz, André, Domaine de la Hêtraie, F-69390 Vernaison (FR)**

(74) Mandataire: **Rochet, Michel et al, ATOCHEM Département Propriété Industrielle Cédex 22, F-92091 Paris la Défense (FR)**

**EP 0 090 712 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne de nouveaux éthènes perfluoroalkylés, leur préparation et leur utilisation, sous forme d'émulsions aqueuses, comme transporteurs d'oxygène, et plus particulièrement comme substituts du sang ou comme agents de perfusion pour la conservation des organes avant transplantation.

Les nouveaux éthènes perfluoroalkylés, objets de l'invention, sont constitués par les isomères trans des bis-(perfluoroalkyl)-1,2-éthènes répondant à la formule suivante:

$$R_F-CH=CH-R_F'$$

dans laquelle les groupes $R_F$ et $R_F'$ représentent des radicaux $C_nF_{2n+1}$, dont l'un au moins est ramifié. La valeur de n va de 3 à 20 pour les groupes ramifiés et de 1 à 20 pour les groupes linéaires.

Les éthènes perfluoroalkylés ne comportant que des chaînes perfluorées linéaires sont connus, notamment par les publications de G. SANTINI, M. LE BLANC et J. G. RIESS, Tetrahedron, 29, 2411 (1973), et de F. JEANNEAUX, G. SANTINI, M. LE BLANC, A. CAMBON et J. G. RIESS, Tetrahedron, 30, 4197 (1974). L'art antérieur ne fait pas état d'éthènes perfluoroalkylés comportant des chaînes ramifiées.

L'utilisation de composés perfluorés ou hautement fluorés comme transporteures des gaz respiratoires dans des substituts du sang a déjà fait l'objet de nombreux travaux. Une revue de ce sujet a été publiée par J. G. RIESS et M. LE BLANC dans Angewandte Chemie, International Edition in English, 17, 621—700 (1978).

Pour qu'un substitut du sang soit considéré comme entièrement satisfaisant, il doit remplir simultanément cinq conditions:
— pouvoir être obtenu à l'état pur et sous une forme parfaitement définie;
— posséder un pouvoir élevé de dissolution des gaz respiratoires;
— être inerte et atoxique;
— être apte à former des émulsions aqueuses stables du type huile dans l'eau, avec une taille moyenne des particules inférieure à 0,1 µm, sans particule supérieure à 0,6 µm;
— pouvoir être éliminé assez rapidement de l'organisme.

Aucun des transporteurs fluorés connus jusqu'ici ne satisfait pleinement à toutes ces conditions. Les bis-(perfluoroalkyl)-1,2-éthènes à chaînes linéaires de l'art antérieur, bien que représentant déjà un notable progrès du point de vue pureté, sont toujours obtenus sous forme de mélanges des deux isomères cis et trans, ce dernier étant prépondérant. C'est ainsi que l'analyse chromatographique du bis-(perfluo-n-hexyl)-1,2-éthène, ou dihydro-7,8-perfluorotétradécène-7, montre qu'il contient 94,1% d'isomère trans et 4,6% d'isomère cis, le reste étant constitué principalement du dérivé saturé correspondant, le bis-(perfluoro-n-hexyl)-1,2-éthane. Les isomères cis et trans de ces bis-(perfluoroalkyl)-1,2-éthènes linéaires ont des ponts d'ébullition suffisamment rapprochés pour que leur séparation par distillation soit difficile, mais cependant suffisamment distincts pour entraîner des différences dans les vitesses d'excrétion.

La demanderesse a découvert, de façon tout-à-fait inattendue, que les réactions habituelles de formation des bis-(perfluoroalkyl)-1,2-éthènes, qui, dans le cas des composés à chaînes perfluorées linéaires, conduisent à des mélanges d'isomères cis et trans, donnent dans la majorité des cas exclusivement l'isomère trans lorsqu'au moins l'un des groupes perfluoroalkyles est ramifié, et en tous cas plus de 99,7% d'isomère trans.

Ces réactions de formation des bis-(perfluoroalkyl)-1,2-éthènes comportent, soit l'addition d'un iodure de perfluoroalkyle $R_FI$ sur un mono-(perfluoroalkyl)-1,2-éthène $R_F'-CH=CH_2$, suivie d'une déshydroioduration par une base, qui peut s'écrire:

$$R_FI + R_F'-CH=CH_2 \longrightarrow R_F-CH_2-CHI-R_F' \xrightarrow{OH^-} R_F-CH=CH-R_F'$$

soit la réaction d'un iodure de perfluoroalkyle $R_FI$ avec un bromo-1-perfluoroalkyl-1-éthène $R_F'-CBr=CH_2$ en présence de cuivre, dans un solvant comme le diméthylformamide, qui peut s'écrire:

$$R_FI + R_F'-CBr=CH_2 \xrightarrow[\text{Solvant}]{\text{Cu}} R_F-CH=CH-R_F'$$

Pour la préparation des composés selon l'invention, la chaîne perfluorée ramifée peut indifféremment être portée par un seul des partenaires des réactions ci-dessus ou par les deux à la fois. Dans tous les cas on obtient exclusivement l'isomère trans du bis-(perfluoroalkyl)-1,2-éthène ou tout au moins un mélange contenant plus de 99,7% d'isomère trans. Les groupes $R_F$ et $R_F'$ peuvent avoir la même condensation en carbone ou une condensation différente.

Les composés selon l'invention ont un domaine liquide très étendu, des tensions de vapeur faibles et des tensions superficielles très basses. Ils ont la même inocuité que les dérivés linéaires correspondants et possèdent un pouvoir dissolvant vis-à-vis de l'oxygène au moins égal. C'est ainsi que 100 ml du perfluoroisopropyl-1-perfluoro-n-hexyl-2-éthène, de formule:

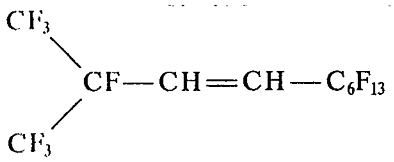

dissolvent jusqu'à 50 ml d'oxygène à 37° C sous une pression de 760 mm de mercure (101,325 kPa).

La demanderesse a également découvert de façon inattendue que ces composés ramifiés, objets de l'invention, se distinguent des composés linéaires correspondants par leur stables et une plus grande vitesse d'élimination de l'organisme.

Ces émulsions très stables et très fines dans l'eau ou dans un sérum physiologique, artificiel ou non, s'obtiennent facilement en utilisant comme agents tensio-actifs des condensats d'oxyde d'éthylène et d'oxyde de propylène, tels que le produit commercialisé par la demanderesse sous la marque déposée PLURONIC F-68, ou des lécithines de jaune d'œuf ou de soja, ou des mélanges de ces composés, ou tout autre tensio-actif adéquat, y compris les tensio-actifs à chaîne fluorée. La dispersion du composé selon l'invention dans l'eau ou dans le sérum physiologique s'effectue par des moyens connus, par exemple à l'aide d'un homogénéisateur sous pression ou sous l'action d'ondes ultra-sonores. Des microémulsions thermodynamiquement stables peuvent également être obtenues en utilisant une combinaison appropriée d'agents tensio-actifs.

Les exemples suivants, donnés à titre non limitatif, illustrent la préparation des composés selon l'invention et la préparation d'émulsions aqueuses contenant ces produits.

## Exemple 1

### Préparation du trans-bis-(perfluoroisopropyl)-1,2-éthène

Dans un autoclave on chauffe à 200° C pendant 16 heures un mélange de 764 g de perfluoroisopropyl-1-éthène (3,90 moles) et de 2370 g d'iodo-1-perfluoroisopropane (8,0 moles). Le mélange réactionnel est refroidi à 0° C puis lavé 3 fois avec une solution aqueuse d'iodure de potassium à 10% en poids. La phase organique est décantée, filtrée sur un papier séparateur de phases et distillée. On recueille d'abord l'excès d'iodo-1-perfluoroisopropane (1250 g) puis une fraction intermédiaire de 120 g constituée par un mélange 50/50 en poids de bis-(perfluoroisopropyl)-1,2-éthène et de bis-(perfluoroisopropyl)-1,2-éthane, et enfin 780 g d'iodo-1-bis-(perfluoroisopropyl)-1,2-éthène pur, passant à 41° C sous 15 mm de mercure (1,999 kPa).

On ajoute lentement 170 g de cet iodo-1-bis-(perfluoroisopropyl)-1,2-éthène (0,34 mole) à une solution de 17,4 g d'hydroxyde de potassium dans 250 ml d'alcool éthylique, refroidie à 0° C. On maintient le mélange sous agitation pendant 1 heure à la température ambiante. On filtre ensuite l'iodure de potassium formé et ajoute 250 ml d'eau. On décante la phase organique, la filtre sur papier séparateur de phase et la distille. On recueille 78 g de trans-bis-(perfluoroisopropyl)-1,2-éthène, bouillant à 80° C sous 760 mm de mercure (101,325 kPa) et chromatographiquement pur.

## Exemple 2

### Préparation du trans-perfluoroisopropyl-1-perfluoro-n-hexyl-2-éthène

Un mélange de 1730 g de perfluoro-n-hexyl-1-éthène (5,0 moles) et de 2960 g d'iodo-1-perfluoroisopropane (10,0 moles) est chauffé pendant 20 heures à 190° C dans un autoclave. Après refroidissement, les produits de la réaction sont lavés par trois fois avec 200 ml d'une solution aqueuse d'iodure de potassium à 10% en poids. La phase organique décantée est filtrée sur un papier séparateur de phases, puis distillée. On recueille successivement 1200 g d'iodo-1-perfluoroisopropane, passant à 25—30° C à la pression atmosphérique, puis, sous pression réduite, 2470 g d'iodo-1-perfluorohexyl-1-perfluoroisopropyl-2-éthane, distillant à 75° C sous 15 mm de mercure (1,999 kPa). Le résidu (430 g) est constitué essentiellement de télomères de formule:

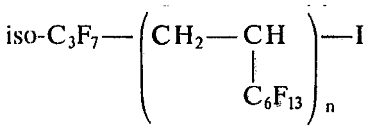

En l'espace de 2 à 3 heures on ajoute goutte à goutte 2026 g d'iodo-1-perfluorohexyl-1-perfluoroisopropyl-2-éthane (3,16 moles) à 221 g d'une solution alcoolique d'hydroxyde de potassium à 10% en poids, agitée et refroidie à 0°C. L'agitation est ensuite maintenue pendant deux heures à la température ambiante. On filtre le précipité d'iodure de potassium, que l'on lave avec 3 fois 100 ml d'éthanol. On ajoute 2 litres d'eau distillée et l'on sépare la phase organique. On la filtre sur papier séparateur de phase et on la destille sous pression réduite. On recueille 1540 g de trans-perfluoroisopropyl-1-perfluoro-n-hexyl-2-éthène, bouillant à 67°C sous 20 mm de mercure (2,666 kPa) et chromatographiquement pur.

## Exemple 3

### Préparation du trans-perfluoroisopropyl-1-perfluoro-n-hexyl-2-éthène

Dans une ampoule scellée de 300 ml on chauffe à 195°C pendant 48 heures un mélange de 5,0 g de perfluoroisopropyl-1-éthène (25,5 millimoles) et de 23,8 g d'iodo-1-perfluorohexane (51,1 millimoles). Le mélange est ensuite ramené à la température ambiante et lavé avec 20 ml d'une solution aqueuse d'iodure de potassium à 10% en poids. La phase perfluorée est décantée, filtrée sur papier séparateur de phases, puis distillée. On recueille à 90°C sous 20 mm de mercure (2,666 kPa) 3,85 g d'iodo-1-perfluoroisopropyl-1-perfluorohexyl-2-éthane.

Ce produit intermédiaire est soumis à une déshydroisoduration par une solution alcoolique d'hydroxyde de potassium dans des conditions identiques à celles utilisées dans l'exemple 2 pour la déshydroioduration de l'isomère iodo-1-perfluorohexyl-1-perfluoroisopropyl-2-éthane. On obtient de nouveau le trans-perfluoroisopropyl-1-perfluoro-n-hexyl-2-éthène chromatographiquement pur.

## Exemple 4

### Préparation d'une émulsion de trans-perfluoroisopropyl-1-perfluoro-n-hexyl-2-éthène

On mélange de 1,5 ml (2,55 g) de trans-perfluoroisopropyl-1-perfluoro-n-hexyl-2-éthène et de 8,5 ml d'une solution aqueuse contenant 0,29 g de PLURONIC F-68 (marque déposée de PCUK pour un copolymère en bloc d'oxyde d'éthylène et d'oxyde de propylène) et 0,043 g de lécithine de jaune d'œuf, est soumis à l'action des ultra-sons dans un appareil SONIFIER B-30 BRANSON muni d'une sonde de 3 mm de diamètre. Après deux irradiations ultra-sonores de 30 secondes, on obtient une émulsion qui passe en totalité au travers d'un filtre MILLIPORE ayant des pores calibrés de 0,45 µm de diamètre. La taille moyenne des particules, mesurée à l'aide d'un appareil NANOSIZER-COULTRONICS, est inférieure à 0,3 µm. Après une semaine de stockage à la température ambiante on n'observe ni sédimentation, ni variation de la taille des particules.

A titre comparatif on réalise dans les mêmes conditions une émulsion aqueuse de bis-(perfluoro-n-butyl)-1,2-éthène. La taille des particules de cette émulsion est analogue à celle de l'émulsion précédente, mais au stockage on observe au bout d'une semaine la formation d'un sédiment.

## Revendications

1. Isomères trans des bis-(perfluoroalkyl)-1,2-éthènes répondant à la formule générale:

$$R_F-CH=CH-R_F'$$

dans laquelle les groupes $R_F$ et $R_F'$ représentent des radicaux $C_nF_{2n+1}$ dont l'un au moins est ramifié et où n va de 3 à 20 pour les groupes ramifiés et de 1 à 20 pour les groupes linéaires.

2. Procédé de préparation des bis-(perfluoroalkyl)-1,2-éthènes ramifiés selon la revendication 1, caractérisé en ce que l'on additionne un iodure de perfluoroalkyle ramifié sur un mono-(perfluoroalkyl)-éthène linéaire ou ramifié et en ce que l'on soumet le dérive iodé obtenu à une déshydroioduration par une base alcaline.

3. Procédé de préparation des bis-(perfluoroalkyl)-1,2-éthènes ramifiés selon la revendication 1, caractérisé en ce que l'on additionne un iodure de perfluoroalkyle linéaire ou ramifié sur un mono-(perfluoroalkyl)-éthène ramifié et en ce que l'on soumet le dérive iodé obtenu à une déshydroioduration par une base alcaline.

4. Utilisation des bis-(perfluoroalkyl)-1,2-éthènes selon la revendication 1, sous forme d'émulsions aqueuses du type huile dans l'eau, comme transporteurs de gaz respiratoires.

**Patentansprüche**

1. trans-Isomere der 1,2-Bis-perfluoralkyl-ethene der allgemeinen Formel

$$R_F-CH=CH-R_F'$$

in der die Gruppen $R_F$ und $R_F'$ $C_nF_{2n+1}$-Radikale bedeuten, von denen wenigstens eines verzweigt ist, und wobei n die Werte von 3 bis 20 für die verzweigten Gruppen und von 1 bis 20 für die geradkettigen Gruppen haben kann.

2. Verfahren zur Herstellung der verzweigten 1,2-Bis-perfluoralkylethene nach Anspruch 1, dadurch gekennzeichnet, daß man ein verzweigtes Perfluoralkyljodid zu einem geradkettigen oder verzweigten Monoperfluoralkylethen zugibt und daß man aus dem erhaltenen Jodderivat mit einer Alkalibase Jodwasserstoff abspaltet.

3. Verfahren zur Herstellung der verzweigten 1,2-Bis-perfluoralkylethene nach Anspruch 1, dadurch gekennzeichnet, daß man ein geradkettiges oder verzweigtes Perfluoralkyljodid zu einem verzweigten Monoperfluoralkylethen zugibt und daß man aus dem erhaltenen Jodderivat mit einer Alkalibase Jodwasserstoff abspaltet.

4. Verwendung der 1,2-Bis-perfluoralkylethene nach Anspruch 1, in Form von wäßrigen Emulsionen des Typs »Öl in Wasser« als Transportmittel für Atmungsgase.

**Claims**

1. Trans-isomers of the 1,2-bis-(perfluoroalkyl)-ethenes corresponding to the general formula:

$$R_F-CH=CH-R_F'$$

in which the groups $R_F$ and $R_F'$ represent $C_nF_{2n+1}$ radicals, of which at least one is branched, and where n ranges from 3 to 20 for branched groups and from 1 to 20 for linear groups.

2. Process for the preparation of branched 1,2-bis-(perfluoroalkyl)-ethenes according to Claim 1, characterized in that an addition reaction is carried out between a branched perfluoroalkyl iodide and a linear or branched mono-(perfluoroalkyl)-ethene and in that the iodine-containing derivative obtained is subjected to a dehydroiodination by means of an alkaline base.

3. Process for the preparation of the branched 1,2-bis-(perfluoroalkyl)-ethenes according to Claim 1, characterized in that an addition reaction is carried out between a linear or branched perfluoroalkyl iodide and a branched mono-(perfluoroalkyl)-ethene and in that the iodine-containing derivative obtained is subjected to a dehydroiodination by means of an alkaline base.

4. Use of the 1,2-bis-(perfluoroalkyl)-ethenes according to Claim 1, in the form of aqueous emulsions of the oil-in-water type, as vehicles for respiratory gases.